# EUROPEAN PATENT SPECIFICATION

(11) **EP 3 740 270 B1**
(45) Date of publication and mention of the grant of the patent: **09.12.2026**
(21) Application number: 19701145.5
(22) Date of filing: 15.01.2019
(51) Int. Cl.: A61M 16/10, A61M 16/16

(54) **SAFETY WARNING SYSTEM**
SICHERHEITSWARNSYSTEM
SYSTÈME D'AVERTISSEMENT DE SÉCURITÉ

(30) Priority: 17.01.2018 GB 201800774
(43) Date of publication of application: 25.11.2020
(73) Proprietor: Medovate Limited, Ely Road Milton Cambridgeshire CB24 6WZ (GB)
(72) Inventor: MARIYASELVAM, Maryanne Zonia Ajanthini, Norfolk PE30 1HY (GB)
(74) Representative: White, Andrew John
(86) International application number: PCT/GB2019/050108
(87) International publication number: WO 2019/141978

(56) References cited:
- US-A1- 2009 078 251
- US-B1- 6 363 930
- US-B2- 7 428 902
- DOYLE ALEXANDER ET AL: "The simultaneous use of a heat and moisture exchanger and a heated humidifier causes critical airway occlusion in less than 24 hours", JOURNAL OF CRITICAL CARE, vol. 30, no. 4, 2015, XP029223209, ISSN: 0883-9441, DOI: 10.1016/J.JCRC.2015.03.033

## Description

### Field of the invention

The present disclosure relates to a safety warning system, device and method for detecting the temperature or humidity in a respiratory circuit, and in particular for detecting the use of a heated humidifier in combination with a heat and moisture exchanger in a respiratory circuit.

### Background

Respiratory circuits in anaesthesia and intensive care may include active heated humidification circuits or passive heat and moisture exchanger (HME) devices. Either can be used but it is important that both are not used together for prolonged periods of time. An alert from the National Health Service in England highlighted the problem in the document of the 15th December 2015 (Alert Reference Number NHS/PSA/W/2015/012) and a peer-reviewed paper (Doyle A, Mariyaselvam M, Wijewardena G, English N, Gent E, Young P. The simultaneous use of a heat and moisture exchanger and a heated humidifier causes critical airway occlusion often in less than 24 hours. Journal of Critical Care; 2015 Aug;30(4):863.e1-3) describes the problem in detail. A life-threatening blockage of the HME can occur if both an HME and heated humidifier are used in the same respiratory circuit.

The HME normally has a chamber and connector at the patient end (patient-end chamber), a porous material and/or a warming and humidifying component centrally and a chamber and connector for the ventilator tubing (ventilator-end chamber).

In normal use, the patient-end chamber and connector of the HME becomes warm and humid from the patient exhaled gases initially inside the chamber and over time this conducts through to the external walls of the chamber. The ventilator-end chamber, in normal use, remains relatively colder than the patient end chamber as the heat and moisture of the exhaled gases are substantially prevented from reaching the ventilator-end chamber by the central heat and moisture exchanging components. The humidity and temperature of the ventilator remains relatively low and the containing gases are repeatedly refreshed with cool and drier gases with each ventilation cycle.

If a heated circuit is applied to the HME in error, the ventilator-end chamber becomes abnormally hot and humidified. The temperature at the ventilator-end chamber of a correctly used HME is commonly at room temperature or below, however if accidently placed into a heated circuit, this temperature will be normally at least 35 degrees Celsius.

The HME devices currently used do not have any indication to the users that the error or using both devices at once has occurred. Belluzzi in US 2009/0205659 A1 describes a device which is a humidity (not temperature) indicator enclosed in a housing to detect humidity in relation to the patient-end chamber.

US 2009/078251 describes a respiratory gas conditioning system wherein a heat and moisture exchanger is located close to a ventilator and a water reservoir is located upstream from the heat and moisture exchanger to moisture-enrich the has exhaled by the patient. US 7428902 describes a humidifier system for heating and humidifying respiratory gases.

### Summary of the invention

Aspects of the invention are as set out in the independent claims and optional features are set out in the dependent claims. Aspects of the invention may be provided in conjunction with each other and features of one aspect may be applied to other aspects.

The present disclosure relates to a safety warning system for detecting the temperature or humidity in a respiratory circuit. In some examples the safety warning system may include a temperature indicating element within the respiratory circuit and visible through a transparent element or applied to the outside of the circuit. The safety warning system may apply the temperature indicating element for example to a heat and moisture exchanger or heat and moisture exchanger filtering device or the respiratory tubing connected to a heat and moisture exchanger.

Embodiments of the disclosure provide a safety warning system comprising a temperature detecting component within the ventilator-end chamber or a temperature detecting component applied to the outside of the ventilator-end chamber and configured to alert a user that the error of using a heat and moisture exchanger in an actively heated and humidified circuit has occurred and may facilitate correction of the error by removal of the heat and moisture exchanger or the heated and humidified circuit.

To illustrate with an example applied in an intensive care unit - a colour changing indicator triggered at 30 degrees Celsius would be one colour or transparent at room temperature (for example 20-25 degrees Celsius) and once above 30 degrees Celsius would be a different colour or transparent - for example a rise above 30 degrees Celsius would occur if a heated humidifier was used with the heat and moisture exchanger raising the temperature of the ventilator-end chamber to approximately 36 degrees Celsius.

One example embodiment uses a temperature sensitive colour change component such as but not limited to, thermochromic pigment or liquid crystal technology as a sticker to the inside or to the outside of the ventilator-end chamber.

Another example embodiment makes use of the application of pigment within the plastic of the ventilator-end chamber.

Another example has a colour change component or series of components indicating different temperatures on or within the patient-end chamber or the central component to indicate the level of heat within the patient-end chamber.

Another example is a colour change component within the ventilator-end chamber which is visible through transparent walls.

Another example provides for the colour change component to reveal advisory wording and/or symbols to alert the user when the ventilator-end chamber is humidified or heated.

Another example uses a temperature probe rather than a colour changing component, within or applied to the outside of the ventilator-end chamber which activates a visual or audible alarm.

Another example uses a moisture or humidity detector within the ventilator-end chamber as a detector.

In accordance with the invention there is provided a safety warning system for detecting the use of a heated humidifier with a heat and moisture exchanger in a respiratory circuit, the system comprising:
a heat and moisture exchanger defining at least a portion of the respiratory circuit, and configured to exchange air between a patient and a ventilator; and
   a temperature indicating element configured to provide an indication that the heat and moisture exchanger is used in combination with a heated humidifier based on the temperature differential between the air exchanged with the patient and the air exchanged with the ventilator in the respiratory circuit
wherein the heat and moisture exchanger comprises a patient-end chamber coupled to a patient-side opening for exchanging air with the patient, and a ventilator-end chamber coupled to at least one ventilator-side opening for exchanging air with the ventilator, wherein the patient-end chamber and ventilator-end chamber are separated by a porous material, and wherein the temperature indicating element is configured to provide an indication that the temperature in the ventilator-end chamber is at and/or above a selected threshold; and
wherein the temperature indicating element is (i) provided inside the ventilator-end chamber, (ii) coupled to an interior wall of the ventilator-end chamber, (iii) coupled to an exterior wall of the ventilator-end chamber, or (iv) coupled to the porous material (131) that separates the patient-end chamber from the ventilator-end chamber (137).

In some embodiments of the invention the heat and moisture exchanger is configured to exchange air between a patient and a ventilator by providing a temperature differential (for example across a porous material that may act as a filter) between air exchanged with the patient and air exchanged with the ventilator.

Providing an indication that the temperature in the ventilator-end chamber is at and/or above a selected threshold may indicate that the temperature differential between the air exchanged with the patient and the air exchanged with the ventilator in the respiratory circuit is not sufficiently great for the air exchanged with the patient to be refreshed by the air exchanged with the ventilator, which in turn means that the humidity in the heat and moisture exchanger becomes too great, leading to an unacceptable buildup of moisture in the heat and moisture exchanger, which in turn may lead to a critical airway occlusion as the respiratory circuit may become blocked by liquid buildup in the heat and moisture exchanger.

In some examples the temperature indicating element is provided as a waterproof and/or laminated tab or disc within the ventilator-end chamber of the heat and moisture exchanger.

The temperature indicating element may be sealed, for example so that it is waterproof. For example. the temperature indicating element may be hermetically sealed from the respiratory circuit. The temperature indicating element may comprise a thermochromic pigment.

In some examples the temperature indicating element is configured to be opaque when the temperature is below a selected threshold, and to be clear to reveal a warning indicating when the temperature is at and/or above a selected threshold. The selected threshold may be at least 30 degrees Celsius.

In some examples the temperature indicating element comprises an electronic temperature sensor configured to send signals indicative of the temperature in at least a portion of the respiratory circuit to a remote device. Additionally or alternatively in some examples the temperature indicating element may comprise means for providing an audible alert, such as a buzzer, in response to the temperature being at and/or above a selected threshold.

In an example of the disclosure there is provided a device for detecting temperature in a heat and moisture exchanger, the device having a temperature indicating element. The temperature indicating element may be adhered or applied to the outside of the heat and moisture exchanger. In other examples the temperature indicating element may be embedded or impregnated within the wall of the heat and moisture exchanger. In some examples the temperature indicating element is within the respiratory circuit or applied to the inner surface of the heat and moisture exchanger. In some examples the temperature indicating element includes an element impregnated with at least one substance that changes colour or becomes transparent with variation in temperature. In some examples the device uses thermochromic pigment as an indicator, and additionally or alternatively in other examples the device uses liquid crystal technology as an indicator. In some examples the device adheres to the heat and moisture exchanger. In some examples the device is within the ventilator end of the heat and moisture exchanger. In other examples the device is in the patient side of the heat and moisture exchanger. In some examples the device is at the filter and/or heat exchange components of the heat and moisture exchanger. The device may reveal symbols or words when the colour change occurs. In some examples the device is an electrical temperature probe which activates an audio or visual alarm. In some examples the device detects moisture causing a colour change.

In some examples the device may comprise a communications interface, a processor and optionally a battery, as well as a temperature sensor. The device may additionally comprise a means for wirelessly powering the device and/or charging the battery. The processor may be programmed to make a determination of the temperature based on signals received from the temperature indicating element (for example from the temperature sensor via the communications interface) and send an indication of the temperature and/or a warning signal to a remote device. For example, the processor may be configured to send a warning signal to a remote device if the signals received from the temperature indicating element indicate that the temperature is at and/or above a selected threshold.

It will be understood that the device described above may be used in combination with the safety warning system described above.

Another example of the disclosure provides a heat and moisture exchanger comprising the device described above.

Another example provides a heat and moisture exchanger configured to provide an indication that the heat and moisture exchanger is used in a respiratory circuit in combination with a heated humidifier;
wherein the heat and moisture exchanger defines at least a portion of the respiratory circuit, and is configured to exchange air between a patient and a ventilator; and
wherein the heat and moisture exchanger comprises a temperature indicating element configured to provide an indication that the temperature in at least a portion of respiratory circuit defined by the heat and moisture exchanger is at and/or above a selected threshold,
and wherein the temperature indicating element is configured to provide an indication that the air exchanged with the ventilator is at or above a selected threshold.

It will be understood that the heat and moisture exchanger may be used with the safety warning system described above.

For example, the heat and moisture exchanger may comprise a patient-end chamber coupled to a patient-side opening for exchanging air with the patient, and a ventilator-end chamber coupled to at least one ventilator-side opening for exchanging air with the ventilator, wherein the patient-end chamber and ventilator-end chamber are separated by a porous material, and wherein the temperature indicating element is configured to provide an indication that the temperature in the ventilator-end chamber is at and/or above a selected threshold.

The temperature indicating element of the heat and moisture exchanger may be provided in the ventilator-end chamber. Alternatively, the temperature indicating element of the heat and moisture exchanger may be coupled to at least one of (i) an interior wall of the ventilator-end chamber and (ii) an exterior wall of the ventilator-end chamber. In other examples the temperature indicating element of the heat and moisture exchanger may be coupled to at least one of (i) the at least one ventilator-side opening and (ii) a tube coupled to the at least one ventilator-side opening. In some examples the temperature indicating element of the heat and moisture exchanger is provided as a laminated tab or disc within the ventilator-end chamber of the heat and moisture exchanger. In some examples the temperature indicating element of the heat and moisture exchanger is impregnated into at least a portion of (i) the material forming the heat and moisture exchanger, (ii) the at least one ventilator-side opening and (iii) the tube coupled to the at least one ventilator-side opening.

The temperature indicating element of the heat and moisture exchanger may be sealed, for example hermetically sealed, from the respiratory circuit. The temperature indicating element of the heat and moisture exchanger may comprise a thermochromic pigment.

In some examples the temperature indicating element of the heat and moisture exchanger is configured to be opaque when the temperature is below a selected threshold, and to be clear to reveal a warning indicating when the temperature is at and/or above a selected threshold. The selected threshold may be at least 30 degrees Celsius.

In some examples the temperature indicating element of the heat and moisture exchanger comprises an electronic temperature sensor configured to send signals indicative of the temperature in at least a portion of the respiratory circuit to a remote device.

In another example of the disclosure there is provided a method of detecting the use of a heated humidifier in a respiratory circuit in combination with a heat and moisture exchanger comprising using a temperature indicating element to indicate that the heat and moisture exchanger is used in combination with a heated humidifier.

The method may further comprise using the temperature indicating element to determine the temperature in at least a portion of the heat and moisture exchanger. The method may further comprise using the temperature indicating element to determine that the temperature in at least a portion of the heat and moisture exchanger is above a selected threshold. The method may further comprise using the temperature indicating element to determine the temperature of air in the heat and moisture exchanger that is exchanged with a ventilator.

In accordance with the invention there is further provided a method of manufacturing a device for use with the safety warning system described above for detecting the use of a heated humidifier apparatus with a heat and moisture exchanger in a respiratory circuit, wherein the heat and moisture exchanger is configured to exchange air between a patient and a ventilator, the method comprising at least one of: (a) impregnating a temperature indicating element into the wall of (i) at least a portion of the heat and moisture exchanger or (ii) a tube in thermal connection with the heat and moisture exchanger; and (b) sealing a temperature indicating element and (i) inserting the sealed temperature indicating element into at least a portion of the heat and moisture exchanger or (ii) coupling the sealed temperature indicating element to the heat and moisture exchanger; wherein the temperature indicating element is configured to provide an indication that the air exchanged with the ventilator is at or above a selected threshold.

### Drawings

Embodiments of the disclosure will now be described, by way of example only, with reference to the accompanying drawings, in which:
Fig. 1 shows an example of a respiratory circuit comprising a heat and moisture exchanger coupled to a ventilator;
Fig. 2 shows the example respiratory circuit of Fig. 1 with an additional heated humidifier coupled to the heat and moisture exchanger;
Fig. 3 shows an example respiratory circuit comprising a safety warning apparatus of embodiments of the claims.

### Specific description

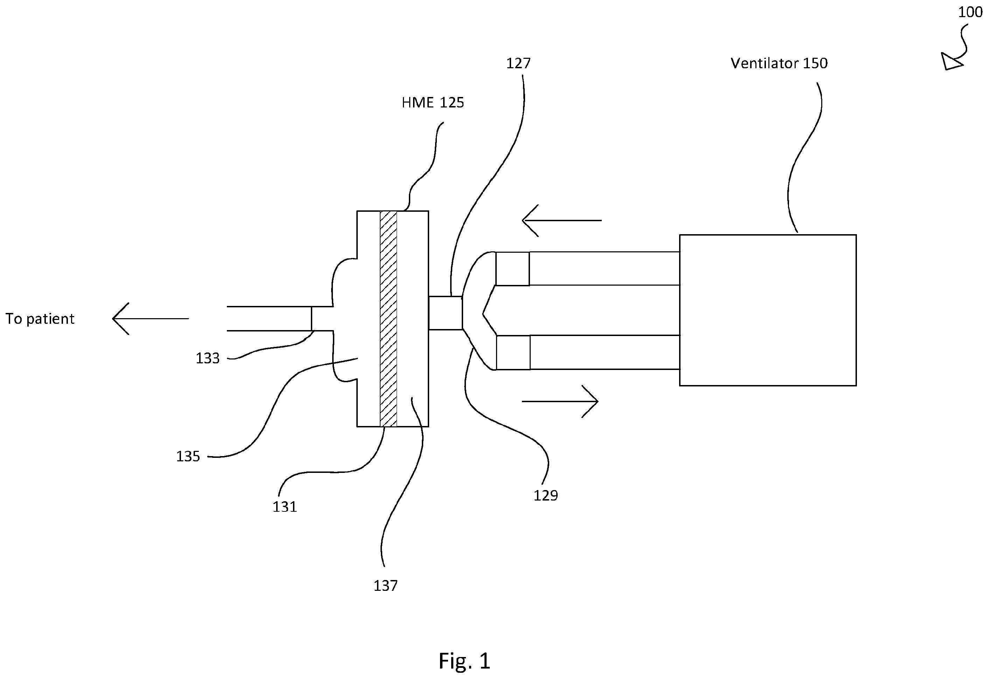

Fig. 1 shows an example respiratory circuit 100 comprising a heat and moisture exchanger 125 coupled to a ventilator 150. The circuit 100 comprises a ventilator 150 coupled to a heat and moisture exchanger 125 via two tubes coupled to a ventilator-side opening 127 of the heat and moisture exchanger 125 via a y-splitter 129. On the other side of the heat and moisture exchanger 125 there is a patient-side opening 133 that is coupled to a tube for insertion into a patient. The heat and moisture exchanger 125 comprises a porous material 131 that separates the heat and moisture exchanger 125 into two chambers; a patient-end chamber 135 and a ventilator-end chamber 137. The ventilator-side opening 127 feeds directly into the ventilator-end chamber 137, and the patient-side opening 133 feeds directly into the patient-end chamber 135.

The heat and moisture exchanger 125 is configured to exchange air with the patient via a tube coupled to the patient-side opening 133. The heat and moisture exchanger 125 is configured to receive oxygenated air from the ventilator 150 along one of the tubes coupled to the ventilator-side opening 127 and to return deoxygenated air from the patient to the ventilator 150 via the other one of the tubes coupled to the ventilator-side opening via the y-splitter 129. The porous material 131 separating the patient-end chamber 135 from the ventilator-end chamber 137 is configured to filter the air passing to and from the patient, and in particular to inhibit any exhaled moisture from reaching the relatively cooler air in the ventilator-end chamber 137.

In some examples, in addition to and/or instead of the porous material 131, the heat and moisture exchanger 125 may comprise heat and moisture exchanging components. The heat and moisture exchanging components may be active (e.g. they may actively work to heat the air) or passive.

In operation, air is exchanged with the patient and the ventilator 150 via the heat and moisture exchanger 125. In use, the patient-end chamber 135, and patient-side opening 133, of the heat and moisture exchanger 125 becomes warm and humid from the patient exhaled gases, initially inside the patient-end chamber 135, and over time this conducts through to the external walls of the patient-end chamber 135. The ventilator-end chamber 137, in normal use, remains relatively colder than the patient-end chamber 135 as the heat and moisture of the exhaled gases are substantially prevented from reaching the ventilator-end chamber 137 by the porous material 131. The humidity and temperature of the ventilator 150 remains relatively low and the containing gases are repeatedly refreshed with cool and drier gases with each ventilation cycle. As such, the heat and moisture exchanger 125 exchanges air between a patient and a ventilator by providing a temperature differential across the porous material 131 between air exchanged with the patient (in the patient-end chamber 135) and air exchanged with the ventilator 150 (in the ventilator-end chamber 137).

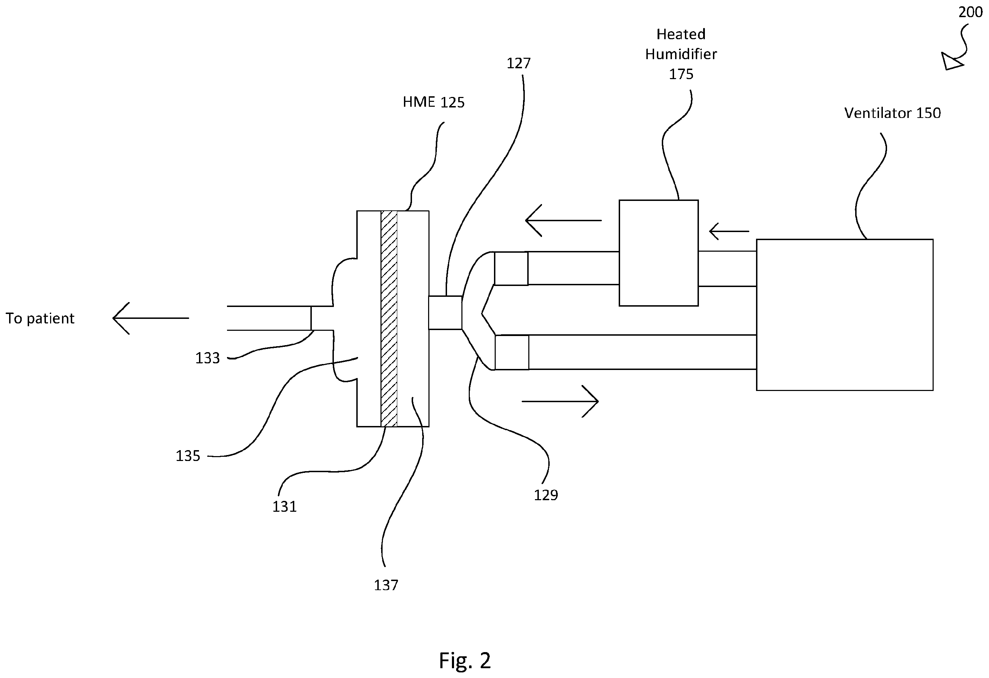

Fig. 2 shows an example respiratory circuit 200, where the example respiratory circuit 100 of Fig. 1 incorporates an additional heated humidifier 175 coupled to the heat and moisture exchanger 125. In this example, the heated humidifier 175 is inserted between the heat and moisture exchanger 125 and the ventilator 150. The heated humidifier 175 is coupled to an output of the ventilator 150 and to the ventilator-side opening 127 of the heat and moisture exchanger 125 via one of the tubes coupled to the y-splitter 129. As such, air passes through the heated humidifier 175 from the ventilator 150 to the heat and moisture exchanger 125.

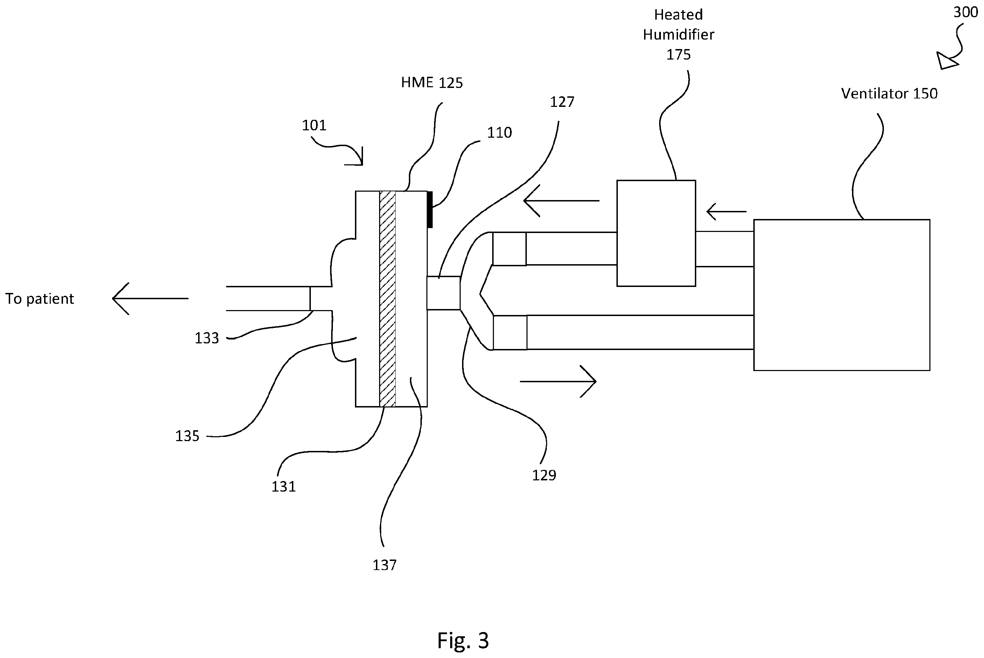

In operation, the heated humidifier 175 heats the air supplied from the ventilator 150 to the heat and moisture exchanger 125. Over time, the ventilator-end chamber 137 therefore becomes abnormally hot and humidified. The temperature at the ventilator-end chamber 137 of a correctly used heat and moisture exchanger 125 is commonly at room temperature or below, however if accidently placed into a heated circuit as shown in Fig. 2 or Fig. 3, this temperature will be normally at least 35 degrees Celsius. As described above, this may lead to a life-threatening blockage of the heat and moisture exchanger 125.

Fig. 3 shows an example respiratory circuit 300 comprising a safety warning system 101 of embodiments of the claims. The safety warning system 101 comprises a heat and moisture exchanger 125, such as the heat and moisture exchanger 125 described above with reference to Figs. 1 and 2, that is configured to exchange air between a patient and a ventilator 150 by providing a temperature differential between air exchanged with the patient and air exchanged with the ventilator 150, and a temperature indicating element 110 configured to provide an indication that the heat and moisture exchanger 125 is used in combination with a heated humidifier 175 based on the temperature differential between the air exchanged with the patient and the air exchanged with the ventilator 150 in the respiratory circuit.

In the example shown in Fig. 3, the heat and moisture exchanger 125 comprises a patient-end chamber 135 coupled to a patient-side opening 133 for exchanging air with the patient, and a ventilator-end chamber 137 coupled to at least one ventilator-side opening 127, 129 for exchanging air with the ventilator 150. The patient-end chamber 135 and ventilator-end chamber 137 are separated by a porous material 131. The heat and moisture exchanger 125 also comprises a temperature indicating element 110, which in the example shown in Fig. 3 is coupled to (for example, adhered to) an exterior wall of the ventilator-end chamber 137, although it will be understood that in other examples the temperature indicating element 110 may be located elsewhere.

A respiratory circuit 300 is therefore defined at least partially by the heat and moisture exchanger 125. The respiratory circuit 300 may be defined between the ventilator 150 along a first tube coupled to the heated humidifier 175 which is coupled in series to the ventilator-side opening 127 via the y-splitter129, through the heat and moisture exchanger 125 via the ventilator-end chamber 137 and the patient-end chamber 135, and then along a tube coupled to patient-side opening 133 to the patient, and then back via the tube coupled to the patient-side opening 133, through the patient-end chamber 135 and then the ventilator-end chamber 137 (via the porous material 131 in the heat and moisture exchanger 125), and then along a second tube coupled to the ventilator-side opening 127 via the y-splitter 129 and back to the ventilator 150.

In the example shown in Fig. 3, because the temperature indicating element 110 is coupled to an exterior wall of the ventilator-end chamber 137 of the heat and moisture exchanger 125, it is outside of the respiratory circuit 300 and therefore may be said to be sealed from the respiratory circuit 300. In the example shown, the temperature indicating element 110 comprises a thermochromic pigment.

The temperature indicating element 110 is configured to provide an indication that the heat and moisture exchanger 125 is used in combination with a heated humidifier such as the heated humidifier 175 shown in Fig. 3. The heat and moisture exchanger 125 may do this based on a temperature differential between the air exchanged with the patient and the air exchanged with the ventilator 150 in the respiratory circuit 300. For example, the temperature indicating element 110 may provide an indication of the temperature differential between the air exchanged with the patient and the air exchanged with the ventilator 150 in the respiratory circuit 300. In the example shown in Fig. 3, the temperature indicating element 110 is configured to provide an indication that the heat and moisture exchanger 125 is used in combination with the heated humidifier 175 by providing an indication that the temperature in the ventilator-end chamber 137 is at and/or above a selected threshold.

In the example shown in Fig. 3, the temperature indicating element 110 is configured to be opaque when the temperature is below a selected threshold, and to be clear to reveal a warning in response to the temperature being at and/or above a selected threshold. The selected threshold may be at least 30 degrees Celsius. The warning may comprise a written warning (such as DANGER or DO NOT USE) and/or symbols that are revealed when the thermochromic pigment becomes clear, for example, and that is readily identifiable by a user such as a clinician.

In operation, air is circulated by the ventilator 150 along the respiratory circuit 300 to exchange oxygenated and deoxygenated air with the patient. The heated humidifier 175 heats the air supplied from the ventilator 150 to the heat and moisture exchanger 125. Over time, the ventilator-end chamber 137 therefore becomes abnormally hot and humidified, and the temperature differential between the air exchanged with the patient in the patient-end chamber 135 and the air exchanged with the ventilator 150 (via the heated humidifier 175) in the ventilator-end chamber 137 reduces, such that the temperature of the air in the patient-end chamber 135 becomes closer to the temperature of the air in the ventilator-end chamber 137. The warm air in the ventilator-end chamber 137 conducts through the wall of the heat and moisture exchanger 125 and to the temperature indicating element 110. The temperature indicating element 110 therefore is warmed up by this conducted heat, and when it reaches a selected threshold (in this example at least 30 degrees Celsius), the thermochromic pigment of the temperature indicating element 110 turns from being opaque to being clear, to reveal a warning (which may be in the form of words, symbols or even just a warning colour) readily identifiable to the user. The user is therefore then prompted to remove the heated humidifier 175 from the respiratory circuit 300.

Although the example shown in Fig. 3 shows the temperature indicating element 110 as being coupled to an external wall of the ventilator-end chamber 137, it may be coupled to other locations on the heat and moisture exchanger 125. It will also be understood that in other examples the temperature indicating element 110 may be provided inside the ventilator-end chamber 137. For example, the temperature indicating element 110 may be provided as a laminated tab or disc within the ventilator-end chamber 137 of the heat and moisture exchanger 125. Additionally or alternatively, in some examples the temperature indicating element 110 may be coupled to an interior wall of the ventilator-end chamber 137 and/or to the porous material 131 that separates the patient-end chamber 135 from the ventilator-end chamber 137. For example, the heat and moisture exchanger 125 may comprise a receiving portion for receiving the temperature indicating element 110. For example, an inside wall of the ventilator-end chamber 137 of the heat and moisture exchanger may comprise a receiving portion, such as a slot, for receiving the temperature indicating element 110. For example, the receiving portion may be configured to receive the temperature indicating element 110 in the form of a tab or disc, such that the temperature indicating element 110 slots into the receiving portion.

In some examples the temperature indicating element 110 may be configured to determine the temperature of air being supplied to the heat and moisture exchanger 125, for example the temperature of air being supplied to the ventilator-end chamber 137 of the heat and moisture exchanger 125. As such, the temperature indicating element 110 may be located in positions where it is in thermal contact with the respiratory circuit, for example in thermal contact with air supplied to the ventilator-end chamber 137 of the heat and moisture exchanger 125. For example, the temperature indicating element 110 may be coupled to at least one of (i) the ventilator-side opening 127, (ii) a tube coupled to the ventilator-side opening 127 and (iii) the porous material 131 that separates the patient-end chamber 135 from the ventilator-end chamber 137. In some examples the temperature indicating element 110 may be impregnated into at least a portion of (i) the material forming the heat and moisture exchanger 125, (ii) the ventilator-side opening 127, (iii) the tube coupled to the at least one ventilator-side opening 127 and (iv) the material forming the porous material 131 that separates the patient-end chamber 135 from the ventilator-end chamber 137.

Although the example shown in Fig. 3 comprises a temperature indicating element 110 that comprises a thermochromic pigment, in other examples the temperature indicating element 110 may comprise an electronic temperature sensor. In such examples the temperature indicating element 110 may be configured to send signals indicative of the temperature in at least a portion of the respiratory circuit to a remote device. As such the temperature indicating element 110 may comprise a communications interface, a processor and optionally a battery, as well as a temperature sensor. The temperature indicating element 110 may additionally comprise a means for wirelessly powering the temperature indicating element 110 and/or charging the battery. The processor may be programmed to make a determination of the temperature based on signals received from the temperature indicating element 110 (for example from the temperature sensor via the communications interface) and send an indication of the temperature and/or a warning signal to a remote device. For example, the processor may be configured to send a warning signal to a remote device if the signals received from the temperature indicating element 110 indicate that the temperature is at and/or above a selected threshold.

A method of detecting the use of a heated humidifier 175 in a respiratory circuit (such as the circuit described above in Fig. 3) in combination with a heat and moisture exchanger 125 is also described herein. The method comprises using a temperature indicating element 110 (such as the one shown in Fig. 3 and as described above) to indicate that a heat and moisture exchanger 125 is used in combination with a heated humidifier 175. In some examples the method further comprises using the temperature indicating element 110 to determine the temperature in at least a portion of the heat and moisture exchanger 125, for example using the temperature indicating element 110 to determine that the temperature in at least a portion of the heat and moisture exchanger 125 is above a selected threshold. In some examples the method further comprises using the temperature indicating element 110 to determine the temperature of air in the heat and moisture exchanger 125 that is exchanged with a ventilator, for example to determine the temperature of air in a ventilator-end chamber 137 of the heat and moisture exchanger 125, as described above with reference to Fig. 3.

As described above, the temperature indicating element 110 shown in Fig. 3 may be impregnated into the wall of (i) at least a portion of the heat and moisture exchanger 125 and/or (ii) a tube in thermal connection with the heat and moisture exchanger 125. As such, a method of manufacturing a device for use with a safety warning system, such as the safety warning system described above with reference to Fig. 3, for detecting the use of a heated humidifier 175 with a heat and moisture exchanger 125 in a respiratory circuit, is also described herein. The method comprises impregnating a temperature indicating element 110 (such as the temperature indicating element 110 described above with reference to Fig. 3) into the wall of (i) at least a portion of the heat and moisture exchanger 125 or (ii) a tube in thermal connection with the heat and moisture exchanger 125.

Also as described above, the temperature indicating element 110 may be sealed (for example by lamination) so that it is waterproof, and either inserted into at least a portion of the heat and moisture exchanger 125 and/or coupled to the heat and moisture exchanger 125. In some examples the temperature indicating element 110 may be hermetically sealed. As such, a method of manufacturing a device for use with a safety warning system, such as the safety warning system described above with reference to Fig. 3, for detecting the use of a heated humidifier 175 with a heat and moisture exchanger 125 in a respiratory circuit, is also described herein. The method comprises sealing a temperature indicating element (such as the temperature indicating element 110 described above with reference to Fig. 3) and (i) inserting the sealed temperature indicating element 110 into at least a portion of the heat and moisture exchanger 125 or (ii) coupling the sealed temperature indicating element 110 to the heat and moisture exchanger 125.

In some examples sealing the temperature indicating element 110 may comprise making the temperature indicating element 110 waterproof and/or hermetically sealing the temperature indicating element 110. Sealing the temperature indicating element 110 may comprise laminating the temperature indicating element 110 into the form of a tab or disc, for example by layering a thermochromic pigment (or optionally a plurality of pigments, such as a thermochromic pigment onto another pigment such that the thermochromic pigment may reveal words or symbols underneath the thermochromic pigment when the temperature is at and/or above a selected threshold) onto a substrate and sealing the substrate carrying the thermochromic pigment (for example by lamination) so that the temperature indicating element 110 is provided as a waterproof thin tab or disc.

It will be understood that the safety warning system 101, and/or the device, described above may be provided in a sealed sterile pack, for example as part of a giving set. For example, the device may be supplied in a sealed sterile pack with a heat and moisture exchanger 125, and optionally tubes for coupling the heat and moisture exchanger 125 to a ventilator and/or a patient. Providing the safety warning system 101 as part of giving set may mean that a user such as a clinician is presented with a single, user-openable, sterile pack that contains many, if not all, of the components necessary for safely ventilating a patient and ensuring that a heated humidifier is not incorrectly used with a heat and moisture exchanger. The single sterile pack may hold the components in a common enclosure, which can be opened to provide access to the components by a single mechanical action, such as tearing or puncturing a wall of that enclosure. The user knows that all of the components in the pack are sterile and safe ready for use, and that they are compatible and will fit together. Providing the giving set may also mean that incorrect or incompatible components are not used incorrectly or by mistake.

In some examples the temperature indicating element 110 may be configured to display an indication to the user that the system is being used correctly. For example, the temperature indicating element 110 may be configured to display a word or symbol (such as SAFE or SAFE TO USE) when the temperature is below the selected threshold, and for the word and/or symbol to disappear when the temperature is at or above the selected threshold. In some examples the temperature indicating element 110 may be configured to display a different word and/or symbol (such as DANGER or DO NOT USE) when the temperature is at and/or above the selected threshold. In such examples the temperature indicating element 110 may therefore be configured to display at least two different indications to the user; a first indication when the temperature is below the selected threshold, and a second indication when the temperature is at and/or above the selected threshold.

It will be appreciated that the air in the patient-end chamber 135 will, in use, generally be at or slightly below body temperature (i.e. at or slightly below 36 to 37 degrees Celsius). As such, it is not necessary for there to be a temperature indicating element 110 in the patient-end chamber 135 as well as the ventilator-end chamber 137 for a temperature differential across the heat and moisture exchanger 125 to be determined - a temperature indicating element 110 provided in or thermal contact with the ventilator-end chamber 137 may be sufficient to provide an indication of the temperature differential across the heat and moisture exchanger 125 if the temperature inside the patient-end chamber 135 is assumed to be around 36 to 37 degrees Celsius. However, it will be understood that in some examples, the safety warning system may comprise a plurality of temperature indicating elements 110. For example, the heat and moisture exchanger may comprise a first temperature indicating element 110 configured to provide an indication of the temperature in the ventilator-end chamber 137 (and optionally to provide an indication if it is at and/or above a selected threshold), and a second temperature indicating element 110 configured to provide an indication of the temperature in the patient-end chamber 135 (and optionally to provide an indication if it is at and/or above a selected threshold, which may be the same threshold or a different threshold as the threshold for the ventilator-end chamber 137).

It will be appreciated from the discussion above that the embodiments shown in the Figures are merely exemplary, and include features which may be generalised, removed or replaced as described herein and as set out in the claims. In the context of the present disclosure other examples and variations of the system, device, apparatus and methods described herein will be apparent to a person of skill in the art.

## Claims

1. A safety warning system for detecting the use of a heated humidifier (175) with a heat and moisture exchanger (125) in a respiratory circuit (100), the system comprising:
a heat and moisture exchanger (125) defining at least a portion of the respiratory circuit, and configured to exchange air between a patient and a ventilator (150); and
a temperature indicating element (110) configured to provide an indication that the heat and moisture exchanger is used in combination with a heated humidifier (175) based on a temperature differential between the air exchanged with the patient and the air exchanged with the ventilator (150) in the respiratory circuit (100);
**characterized in that** the heat and moisture exchanger (125) comprises a patient-end chamber coupled to a patient-side opening (133) for exchanging air with the patient, and a ventilator-end chamber (137) coupled to at least one ventilator-side opening (127) for exchanging air with the ventilator, wherein the patient-end chamber and ventilator-end chamber (137) are separated by a porous material (131), and wherein the temperature indicating element (110) is configured to provide an indication that the temperature in the ventilator-end chamber (137) is at and/or above a selected threshold; and
wherein the temperature indicating element (110) is (i) provided inside the ventilator-end chamber (137), (ii) coupled to an interior wall of the ventilator-end chamber (137), (iii) coupled to an exterior wall of the ventilator-end chamber (137), or (iv) coupled to the porous material (131) that separates the patient-end chamber from the ventilator-end chamber (137).

2. The safety warning system of any of the previous claims wherein the temperature indicating element (110) is sealed from the respiratory circuit (100).

3. The safety warning system of any of the previous claims wherein the temperature indicating element (110) comprises a thermochromic pigment.

4. The safety warning system of any of the previous claims wherein the temperature indicating element (110) is configured to be opaque when the temperature is below a selected threshold, and to be clear to reveal a warning when the temperature is at or above a selected threshold.

5. The safety warning system of claim 4 wherein the selected threshold is at least 30 degrees Celsius.

6. The safety warning system of claim 1 or 2 wherein the temperature indicating element (110) comprises an electronic temperature sensor configured to send signals indicative of the temperature in at least a portion of the respiratory circuit to a remote device.

7. The safety warning system of any of the previous claims wherein the temperature indicating element (110) is provided as a laminated tab or disc within the ventilator-end chamber (137) of the heat and moisture exchanger (125).

8. The safety warning system of any of the previous claims, wherein the temperature indicating element (110) is impregnated into at least a portion of (i) the material forming the heat and moisture exchanger (125), (ii) the at least one ventilator-side opening (127), (iii) a tube coupled to the at least one ventilator-side opening (127) and (iv) the porous material (131) that separates the patient-end chamber from the ventilator-end chamber (137).

9. A method of manufacturing a device for use with the safety warning system of any of claims 1 to 8 for detecting the use of a heated humidifier (175) with a heat and moisture exchanger (125) in a respiratory circuit (100), wherein the heat and moisture exchanger (125) is configured to exchange air between a patient and a ventilator (150), the method comprising at least one of:
(a) impregnating a temperature indicating element (110) into the wall of (i) at least a portion of the heat and moisture exchanger (125) or (ii) a tube in thermal connection with the heat and moisture exchanger (125); and
(b) sealing a temperature indicating element (110) and (i) inserting the sealed temperature indicating element (110) into at least a portion of the heat and moisture exchanger (125) or (ii) coupling the sealed temperature indicating element (110) to the heat and moisture exchanger (125);
wherein the temperature indicating element (110) is configured to provide an indication that the air exchanged with the ventilator (150) is at or above a selected threshold.

## Patentansprüche

1. Sicherheitswarnsystem zum Erfassen der Verwendung eines beheizten Befeuchters (175) mit einem Wärme- und Feuchtigkeitstauscher (125) in einem Atemkreislauf (100), wobei das System Folgendes umfasst:
einen Wärme- und Feuchtigkeitstauscher (125), der mindestens einen Teil des Atemkreislaufs definiert und dazu konfiguriert ist, Luft zwischen einem Patienten und einem Beatmungsgerät (150) auszutauschen; und
ein Temperaturangabeelement (110), das dazu konfiguriert ist, basierend auf einer Temperaturdifferenz zwischen der mit dem Patienten ausgetauschten Luft und der mit dem Beatmungsgerät (150) im Atemkreislauf (100) ausgetauschten Luft eine Angabe bereitzustellen, dass der Wärme- und Feuchtigkeitstauscher in Kombination mit einem beheizten Befeuchter (175) verwendet wird;
**dadurch gekennzeichnet, dass** der Wärme- und Feuchtigkeitstauscher (125) eine patientenseitige Kammer, die mit einer patientenseitigen Öffnung (133) zum Austauschen von Luft mit dem Patienten gekoppelt ist, und einer beatmungsgerätseitigen Kammer (137), die mit mindestens einer beatmungsgerätseitigen Öffnung (127) zum Austauschen von Luft mit dem Beatmungsgerät gekoppelt ist, umfasst, wobei die patientenseitige Kammer und die beatmungsgerätseitige Kammer (137) durch ein poriges Material (131) voneinander getrennt sind, und wobei das Temperaturangabeelement (110) dazu konfiguriert ist, eine Angabe bereitzustellen, dass die Temperatur in der beatmungsgerätseitigen Kammer (137) einen ausgewählten Schwellenwert beträgt und/oder überschreitet; und
wobei das Temperaturangabeelement (110) (i) innerhalb der beatmungsgerätseitigen Kammer (137) bereitgestellt ist, (ii) mit einer Innenwand der beatmungsgerätseitigen Kammer (137) gekoppelt ist, (iii) mit einer Außenwand der beatmungsgerätseitigen Kammer (137) gekoppelt ist oder (iv) mit dem porigen Material (131), das die patientenseitige Kammer von der beatmungsgerätseitigen Kammer (137) trennt, gekoppelt ist.

2. Sicherheitswarnsystem nach einem der vorherigen Ansprüche, wobei das Temperaturangabeelement (110) gegen den Atemkreislauf (100) abgedichtet ist.

3. Sicherheitswarnsystem nach einem der vorherigen Ansprüche, wobei das Temperaturangabeelement (110) ein thermochromes Pigment umfasst.

4. Sicherheitswarnsystem nach einem der vorherigen Ansprüche, wobei das Temperaturangabeelement (110) dazu konfiguriert ist, undurchsichtig zu sein, wenn die Temperatur unter einem ausgewählten Schwellenwert liegt, und durchsichtig zu sein, um eine Warnung zu enthüllen, wenn die Temperatur an oder über einem ausgewählten Schwellenwert liegt.

5. Sicherheitswarnsystem nach Anspruch 4, wobei der ausgewählte Schwellenwert mindestens 30 Grad Celsius beträgt.

6. Sicherheitswarnsystem nach Anspruch 1 oder 2, wobei das Temperaturangabeelement (110) einen elektronischen Temperatursensor umfasst, der dazu konfiguriert ist, Signale, die die Temperatur in mindestens einem Teil des Atemkreislaufs angeben, an eine ortsferne Vorrichtung zu senden.

7. Sicherheitswarnsystem nach einem der vorherigen Ansprüche, wobei das Temperaturangabeelement (110) als eine laminierte Lasche oder Scheibe innerhalb der beatmungsgerätseitigen Kammer (137) des Wärme- und Feuchtigkeitstauschers (125) bereitgestellt ist.

8. Sicherheitswarnsystem nach einem der vorherigen Ansprüche, wobei das Temperaturangabeelement (110) in mindestens einen Teil (i) des Materials, das den Wärme- und Feuchtigkeitstauscher (125) bildet, (ii) der mindestens einen beatmungsgerätseitigen Öffnung (127), (iii) eines Rohrs, das mit der mindestens einen beatmungsgerätseitigen Öffnung (127) gekoppelt ist, und (iv) des porösen Materials (131), das die patientenseitige Kammer von der beatmungsgerätseitigen Kammer (137) trennt, imprägniert ist.

9. Verfahren zum Herstellen einer Vorrichtung zur Verwendung mit dem Sicherheitswarnsystem nach einem der Ansprüche 1 bis 8 zum Erfassen der Verwendung eines beheizten Befeuchters (175) mit einem Wärme- und Feuchtigkeitstauscher (125) in einem Atemkreislauf (100), wobei der Wärme- und Feuchtigkeitstauscher (125) dazu konfiguriert ist, Luft zwischen einem Patienten und einem Beatmungsgerät (150) auszutauschen, wobei das Verfahren mindestens eines der Folgenden umfasst:
(a) Imprägnieren eines Temperaturangabeelements (110) in die Wand (i) mindestens eines Teils des Wärme- und Feuchtigkeitstauschers (125) oder (ii) eines Rohrs in der Wärmeverbindung mit dem Wärme- und Feuchtigkeitstauscher (125); und
(b) Abdichten eines Temperaturabgabeelements (110) und (i) Einsetzen des abgedichteten Temperaturangabeelements (110) in mindestens einen Abschnitt des Wärme- und Feuchtigkeitstauschers (125) oder (ii) Koppeln des abgedichteten Temperaturangabeelements (110) mit dem Wärme- und Feuchtigkeitstauscher (125);
wobei das Temperaturangabeelement (110) dazu konfiguriert ist, eine Angabe bereitzustellen, dass die mit dem Beatmungsgerät (150) ausgetauschte Luft an oder über einem ausgewählten Schwellenwert liegt.

## Revendications

1. Système d'avertissement de sécurité permettant de détecter l'utilisation d'un humidificateur chauffé (175) avec un échangeur de chaleur et d'humidité (125) dans un circuit respiratoire (100), le système comprenant :
un échangeur de chaleur et d'humidité (125) définissant au moins une partie du circuit respiratoire, et conçu pour échanger de l'air entre un patient et un ventilateur (150) ; et
un élément d'indication de température (110) conçu pour fournir une indication que l'échangeur de chaleur et d'humidité est utilisé en combinaison avec un humidificateur chauffé (175) sur la base d'un différentiel de température entre l'air échangé avec le patient et l'air échangé avec le ventilateur (150) dans le circuit respiratoire (100) ;
**caractérisé en ce que** l'échangeur de chaleur et d'humidité (125) comprend une chambre à l'extrémité située côté patient accouplée à une ouverture côté patient (133) pour échanger de l'air avec le patient, et une chambre à l'extrémité située côté ventilateur (137) accouplée à au moins une ouverture côté ventilateur (127) pour échanger de l'air avec le ventilateur, dans lequel la chambre à l'extrémité située côté patient et la chambre à l'extrémité située côté ventilateur (137) sont séparées par un matériau poreux (131), et dans lequel l'élément d'indication de température (110) est conçu pour fournir une indication que la température dans la chambre à l'extrémité située côté ventilateur (137) est à un seuil sélectionné et/ou au-dessus de celui-ci ; et
dans lequel l'élément d'indication de température (110) est (i) prévu à l'intérieur de la chambre à l'extrémité située côté ventilateur (137), (ii) accouplé à une paroi intérieure de la chambre à l'extrémité située côté ventilateur (137), (iii) accouplé à une paroi extérieure de la chambre à l'extrémité située côté ventilateur (137), ou (iv) accouplé au matériau poreux (131) qui sépare la chambre à l'extrémité située côté patient de la chambre à l'extrémité située côté ventilateur (137).

2. Système d'avertissement de sécurité selon n'importe laquelle des revendications précédentes, dans lequel l'élément d'indication de température (110) est étanchéifié par rapport au circuit respiratoire (100).

3. Système d'avertissement de sécurité selon n'importe laquelle des revendications précédentes, dans lequel l'élément d'indication de température (110) comprend un pigment thermochromique.

4. Système d'avertissement de sécurité selon n'importe laquelle des revendications précédentes, dans lequel l'élément d'indication de température (110) est conçu pour être opaque lorsque la température est en dessous d'un seuil sélectionné, et pour être clair afin de révéler un avertissement lorsque la température est à un seuil sélectionné ou au-dessus de celui-ci.

5. Système d'avertissement de sécurité selon la revendication 4, dans lequel le seuil sélectionné est d'au moins 30 degrés Celsius.

6. Système d'avertissement de sécurité selon la revendication 1 ou 2, dans lequel l'élément d'indication de température (110) comprend un capteur de température électronique conçu pour envoyer à un dispositif éloigné des signaux indiquant la température dans au moins une partie du circuit respiratoire.

7. Système d'avertissement de sécurité selon n'importe laquelle des revendications précédentes, dans lequel l'élément d'indication de température (110) est fourni sous forme de languette ou disque stratifié au sein de la chambre à l'extrémité située côté ventilateur (137) de l'échangeur de chaleur et d'humidité (125).

8. Système d'avertissement de sécurité selon n'importe laquelle des revendications précédentes, dans lequel l'élément d'indication de température (110) est imprégné dans au moins une partie (i) du matériau formant l'échangeur de chaleur et d'humidité (125), (ii) de l'au moins une ouverture côté ventilateur (127), (iii) d'un tube accouplé à l'au moins une ouverture côté ventilateur (127) et (iv) du matériau poreux (131) qui sépare la chambre à l'extrémité située côté patient de la chambre à l'extrémité située côté ventilateur (137).

9. Procédé de fabrication d'un dispositif destiné à être utilisé avec le système d'avertissement de sécurité selon n'importe laquelle des revendications 1 à 8 permettant de détecter l'utilisation d'un humidificateur chauffé (175) avec un échangeur de chaleur et d'humidité (125) dans un circuit respiratoire (100), dans lequel l'échangeur de chaleur et d'humidité (125) est conçu pour échanger de l'air entre un patient et un ventilateur (150), le procédé comprenant au moins l'une des étapes suivantes :
(a) l'imprégnation d'un élément d'indication de température (110) dans la paroi (i) d'au moins une partie de l'échangeur de chaleur et d'humidité (125) ou (ii) d'un tube en liaison thermique avec l'échangeur de chaleur et d'humidité (125) ; et
(b) l'étanchéification d'un élément d'indication de température (110) et (i) l'insertion de l'élément d'indication de température étanchéifié (110) dans au moins une partie de l'échangeur de chaleur et d'humidité (125) ou (ii) l'accouplement de l'élément d'indication de température étanchéifié (110) à l'échangeur de chaleur et d'humidité (125) ;
dans lequel l'élément d'indication de température (110) est conçu pour fournir une indication que l'air échangé avec le ventilateur (150) est à un seuil sélectionné ou au-dessus de celui-ci.
